# FASCICULE DE BREVET EUROPEEN

(11) **EP 3 159 237 B1**
(45) Date de publication et mention de la délivrance du brevet: **02.12.2020**
(21) Numéro de dépôt: 16195149.6
(22) Date de dépôt: 21.10.2016
(51) Int. Cl.: B61D 1/06, B61F 3/12

(54) **VÉHICULE FERROVIAIRE COMPRENANT AU MOINS UN BOGIE ABAISSÉ**
SCHIENENFAHRZEUG MIT MINDESTENS EIN ABGESENKTES DREHGESTELL
RAILWAY VEHICLE HAVING AT LEAST A LOWERED BOGIE

(30) Priorité: 23.10.2015 FR 1560142
(43) Date de publication de la demande: 26.04.2017
(73) Titulaire: ALSTOM Transport Technologies, 93400 Saint-Ouen (FR)
(72) Inventeur: Sanchez, Carlos, 75017 Paris (FR); Rodet, Alain, 71100 Chalon Saône (FR); Muyo, Jose Julio, 75015 Paris (FR); Lafoix, Emmanuel, 92400 Courbevoie (FR)
(74) Mandataire: Lavoix

(56) Documents cités:
- EP-A1- 1 270 359
- EP-A2- 0 831 003
- DE-U1- 29 512 446
- US-A- 3 570 408

## Description

La présente invention concerne un véhicule ferroviaire du type comprenant au moins deux voitures adjacentes, comprenant chacune une caisse de voiture, lesdites caisses étant supportées par un bogie commun et étant articulées l'une à l'autre par un dispositif d'articulation fixé aux deux caisses de sorte à former une liaison articulée entre les deux caisses.

L'invention s'applique plus particulièrement aux véhicules ferroviaires à deux étages destinés à des trajets entre plusieurs villes, du type trains à grande vitesse, trains interrégionaux et autres.

Le document EP 0 831 003 décrit un bogie pour un véhicule ferroviaire.

Dans ce type de véhicules ferroviaires, l'interconnexion entre les voitures successives se fait généralement sur un seul niveau, c'est-à-dire que les passagers doivent monter au niveau supérieur ou descendre au niveau inférieur pour passer d'une voiture à la suivante, ce qui complique la circulation dans le véhicule ferroviaire et pose problème pour les personnes à mobilité réduite.

Il a été proposé, par exemple dans le document EP-1 312 526, des véhicules ferroviaires dans lesquels le passage entre deux voitures successives se fait sur les deux niveaux. Cependant, dans ce document, l'interconnexion passe entre deux roues reliées directement à la caisse du véhicule ferroviaire et non au droit d'un bogie supportant les extrémités de deux caisses successives et permettant l'entraînement du véhicule ferroviaire. Dans ce document, lorsqu'un tel bogie est prévu, pour les voitures d'extrémités, le plancher est surélevé pour passer au-dessus du bogie, le véhicule ne comprenant qu'un seul niveau au droit du bogie.

L'interconnexion décrite dans le document EP-1 312 526 n'est donc pas satisfaisante pour les véhicules ferroviaires roulant à vitesse élevée dans lesquels les extrémités de deux voitures successives sont supportées par un bogie. En outre, le véhicule ferroviaire n'est pas optimisé en termes de capacité d'accueil de voyageur.

L'un des buts de l'invention est de pallier ces inconvénients en proposant un véhicule ferroviaire dont deux voitures successives sont supportées par un bogie commun tout en permettant une interconnexion à deux niveaux sans surélévation du plancher.

A cet effet, l'invention concerne un véhicule ferroviaire selon la revendication 1.

Le bogie du véhicule ferroviaire selon l'invention supporte les extrémités de deux voitures adjacentes et est apte à entraîner ces voitures lorsque le train est en circulation grâce au contact entre le dispositif d'articulation et les surfaces de butées. En outre, l'espace prévu dans le bogie pour la réception du dispositif d'articulation permet à une interconnexion à deux niveaux de passer au droit du bogie sans nécessiter de surélever le plancher au droit du bogie. Ainsi, la circulation dans le véhicule ferroviaire peut se faire sans changer de niveau et sans franchir de marches. En outre, le véhicule ferroviaire peut être optimisé en termes de capacité d'accueil de voyageurs.

Selon d'autres caractéristiques du véhicule ferroviaire selon l'invention, prises isolément ou selon toutes les combinaisons techniquement possibles :
- le dispositif d'articulation comprend un premier élément d'articulation fixé à l'une des caisses et un deuxième élément d'articulation fixé à l'autre caisse, lesdits premier et deuxième éléments d'articulation étant reliés l'un à l'autre de sorte à former une liaison articulée entre les deux caisses, le premier élément d'articulation s'étendant en regard de la première surface de butée et le deuxième élément d'articulation s'étendant en regard de la deuxième surface de butée ;
- le bogie comprend un châssis reposant sur quatre roues, une paire de roues s'étendant de chaque côté du châssis, ledit châssis supportant au moins un moteur disposé sur un côté du châssis et relié à au moins une roue par des moyens de transmission, chaque roue comportant un arbre de roue individuel ;
- le bogie comprend deux moteurs disposés chacun sur un des côtés du châssis de part et d'autre de l'espace selon une direction transversale sensiblement perpendiculaire à la direction longitudinale, chaque moteur étant relié à deux des roues du bogie par des moyens de transmission ;
- ledit châssis du bogie comprend deux longerons longitudinaux disposés à l'extérieur des roues et reliés entre eux par des traverses possédant une partie centrale abaissée s'étendant au-dessous du plan défini par l'axe des roues ;
- les planchers inférieurs des voitures et du passage d'interconnexion s'étendent sensiblement à la même hauteur et les planchers supérieurs des voitures et du passage d'interconnexion s'étendent sensiblement à la même hauteur, supérieure à la hauteur des planchers inférieurs ;
- le plan des planchers inférieurs est confondu avec un plan traversant le bogie.

D'autres aspects et avantages de l'invention apparaîtront à la lecture de la description qui suit, donnée à titre d'exemple et faite en référence aux dessins annexés, dans lesquels :
- la figure 1 est une représentation schématique en perspective d'un bogie et d'un dispositif d'articulation d'un véhicule ferroviaire selon l'invention,
- la figure 2 est une représentation schématique en perspective du bogie de la figure 1 supportant une caisse de voiture d'un véhicule ferroviaire selon l'invention,
- la figure 3 est une vue agrandie d'une partie de la figure 2,
- la figure 4 est une représentation schématique en perspective du dispositif d'articulation et des surfaces de butée de la figure 1,
- la figure 5 est une représentation schématique en coupe de l'interconnexion prévue entre les deux voitures adjacentes du véhicule ferroviaire selon l'invention,
- la figure 6 est une représentation schématique de côté d'un véhicule ferroviaire selon l'invention, et
- la figure 7 est une représentation schématique en coupe du véhicule ferroviaire de la figure 6.

Dans la description, les termes « sur », « sous », « au-dessus », « en-dessous » sont définis par rapport à une direction d'élévation d'un véhicule ferroviaire lorsqu'il est disposé sur des rails, c'est-à-dire une direction sensiblement verticale lorsque le train circule. La direction longitudinale est définie par la direction de circulation du véhicule ferroviaire et la direction transversale est la direction sensiblement perpendiculaire à la direction longitudinale et à la direction d'élévation du véhicule ferroviaire.

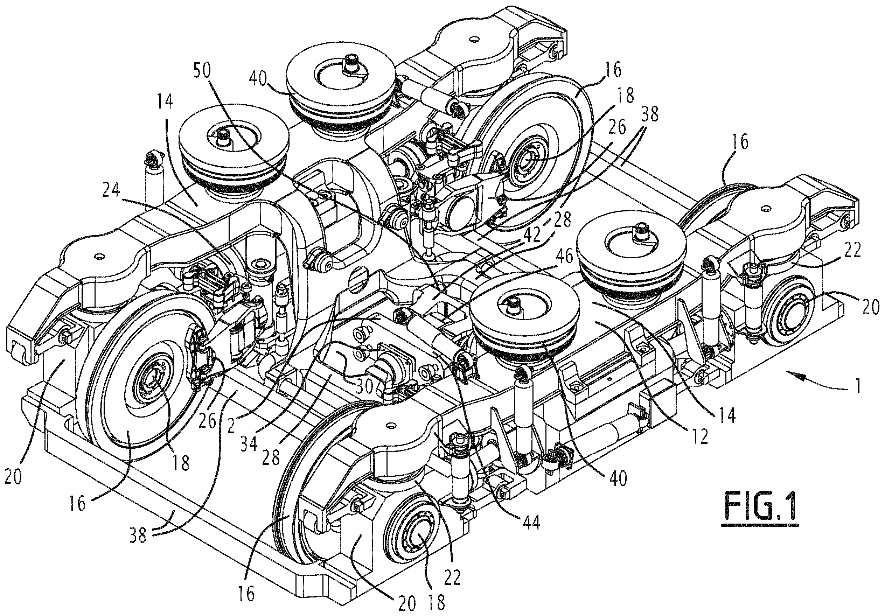

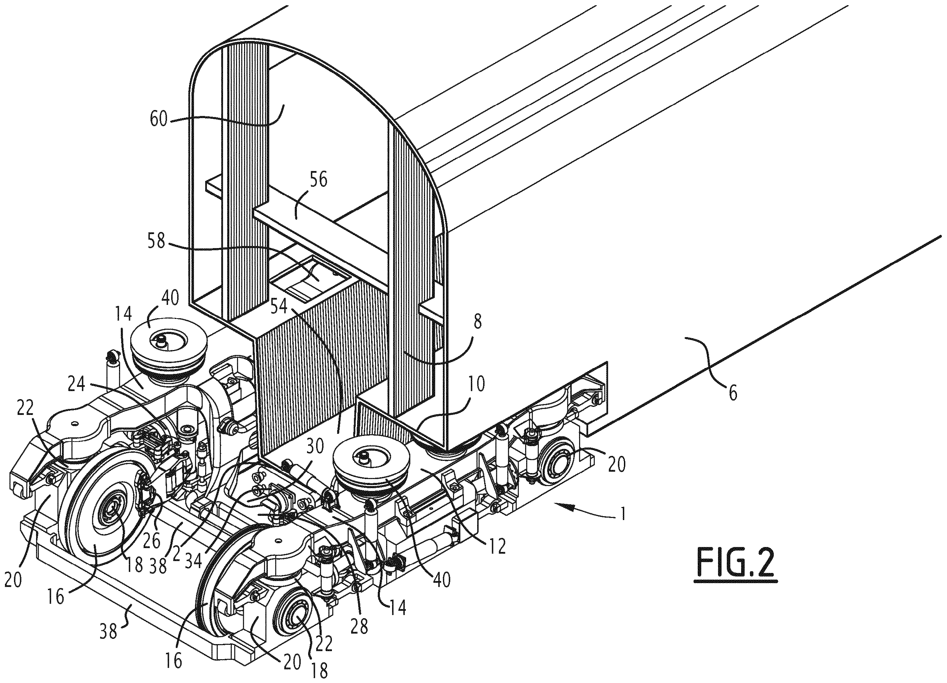

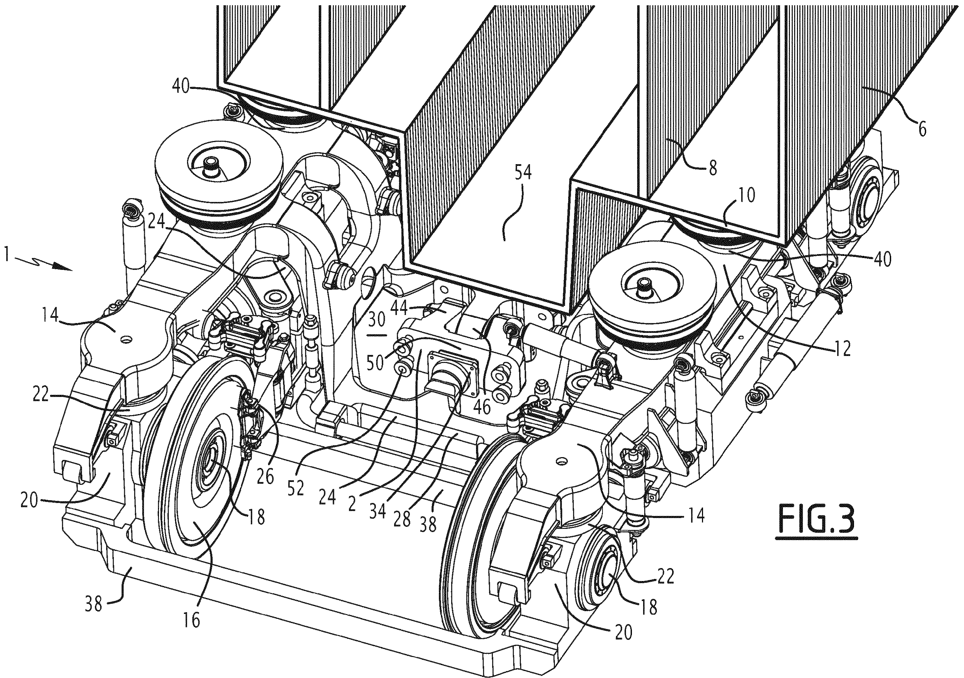

En référence aux figures 1 à 4, on décrit un bogie 1 et un dispositif d'articulation 2 d'un véhicule ferroviaire 4 comprenant au moins deux voitures 6 adjacentes en vue de former un véhicule ferroviaire à deux niveaux dont l'interconnexion permet une circulation sur les deux niveaux, comme cela sera décrit ultérieurement.

Le bogie 1 s'étend sous et entre deux voitures 6 adjacentes et est agencé pour supporter les caisses 8 de ces voitures. Plus particulièrement, le bogie 1 est agencé pour supporter les extrémités 10, en regard l'une de l'autre, des caisses 8 des voitures 6 adjacentes.

Selon l'exemple de réalisation représenté sur la figure 1, le bogie 1 comprend un châssis 12 comprenant deux longerons longitudinaux 14 s'étendant sensiblement selon la direction longitudinale de circulation du véhicule ferroviaire et espacés l'un de l'autre selon la direction transversale sensiblement perpendiculaire à la direction longitudinale et à la direction d'élévation du véhicule ferroviaire.

Deux roues 16 sont montées sur chaque longeron 14, à chacune des parties extrêmes longitudinales de celui-ci, de sorte qu'une paire de roues 16 s'étend de chaque côté du châssis 12. Plus particulièrement, chaque roue 16 comprend un arbre individuel 18 monté en rotation autour d'un axe transversal sur une boîte d'essieu 20. Par arbre individuel, on entend que chaque roue 16 est mobile en rotation par rapport au châssis indépendamment des autres, à la différence d'un bogie traditionnel dans lequel un essieu relie généralement les roues deux à deux et entraîne simultanément deux roues en rotation. Chaque partie extrême d'un longeron longitudinal 14 repose sur une boîte d'essieu 20 par l'intermédiaire d'une suspension primaire 22 autorisant un débattement vertical du châssis 1 par rapport aux roues 16. Les roues sont montées de sorte à s'étendre, selon la direction transversale, à l'intérieur du gabarit défini par le contour du châssis 12, les boîtes d'essieu 20 étant disposées du côté extérieur des roues 16.

Dans le cas d'un bogie 1 motorisé, chaque longeron 14 porte en outre au moins un moteur 24 agencé pour entraîner en rotation les deux roues 16 portées par le longeron. Le moteur est ainsi relié à la boîte d'essieu 20 de chaque roue 16 par des moyens de transmission. Ces moyens de transmission sont par exemple du même type que ceux décrits dans le document EP-1 270 359 et ne seront pas décrits plus en détail ici. Chaque moteur 24 est porté par son longeron 14 entre les deux roues 16 portées par ledit longeron 14.

Le bogie 1 comprend en outre un dispositif de freinage comprenant quatre pinces de frein 26, disposées chacune, autour d'une partie d'une des roues 16 de sorte à freiner les roues 16 de façon connue lorsque le dispositif de freinage est actionné. Les pinces de frein 26 sont chacune disposée du côté intérieur des roues 16 selon la direction longitudinale, c'est-à-dire qu'elles s'étendent vers le centre du bogie 1 selon la direction longitudinale et non vers l'extérieur, comme cela est habituellement le cas. Ainsi, les pinces de frein 26 s'étendent deux à deux l'une en regard de l'autre selon la direction de circulation de sorte que chaque pince 16 s'étend entre les roues d'une des paires de roues selon la direction longitudinale.

Les longerons latéraux 14 sont reliés l'un à l'autre par au moins une traverse 28 s'étendant selon la direction transversale entre les deux longerons 14. Selon le mode de réalisation représenté sur les figures, les longerons 14 sont reliés par deux traverses 28 espacées l'une de l'autre selon la direction longitudinale. Les traverses 28 comprennent au moins une partie centrale s'étendant dans un plan abaissé entre les longerons longitudinaux, c'est-à-dire dans un plan s'étendant sous le plan défini par les axes des roues 16.

Un espace 30 est délimité, dans le plan des traverses 28, par les deux traverses 28 selon la direction longitudinale et par les longerons longitudinaux 14 selon la direction transversale. L'espace 30 s'étend par exemple entre les deux moteurs 24 selon la direction transversale.

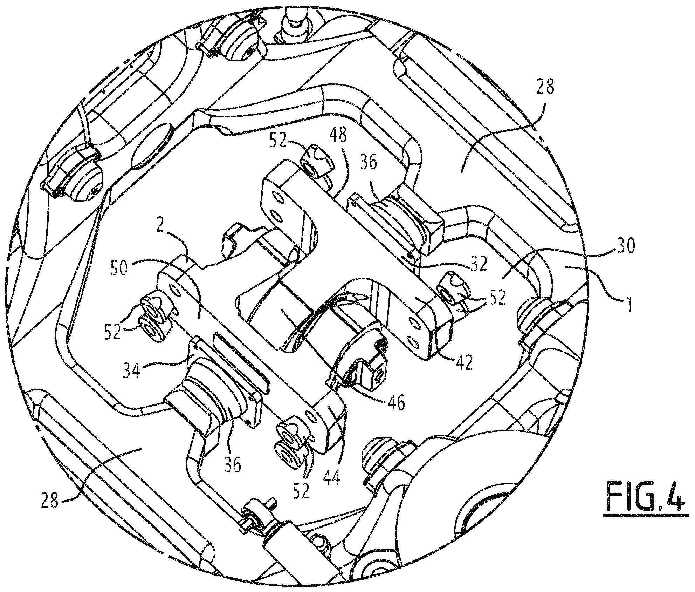

Le bogie 1 comprend en outre une première surface de butée 32 et une deuxième surface de butée 34 s'étendant dans l'espace 30, de part et d'autre de celui-ci selon la direction longitudinale. Les surfaces de butées 32 et 34 s'étendent ainsi l'une en regard de l'autre de part et d'autre de l'espace 30 selon la direction longitudinale, par exemple sensiblement au centre du bogie 1 selon la direction transversale. Les première et deuxième surfaces de butée 32 et 34 sont par exemple portées chacune par une des traverses 28 délimitant l'espace 30, comme cela est plus particulièrement visible sur la figure 4. Les première et deuxième surfaces de butées 32 et 34 sont par exemple formées par des patins s'étendant chacun dans un plan défini par la direction transversale et par la direction d'élévation. Selon le mode de réalisation représenté sur les figures, un élément d'absorption, par exemple en caoutchouc, 36 est disposé entre chaque surface de butées 32, 34 et la traverse 28 qui porte cette surface de butée 32, 34.

Selon le mode de réalisation représenté sur les figures, le bogie 1 comprend en outre des traverses secondaires 38 reliant entre elles les boîtes d'essieu 20, ces traverses secondaires 38 étant disposées de part et d'autre de chaque roue 16, à une hauteur sensiblement identique à celle des traverses 28, c'est-à-dire une hauteur abaissée sous le plan défini par les arbres 18 des roues 16. Ces traverses secondaires 38 assurent le maintien de l'écartement et du parallélisme entre les roues 16.

Le bogie ci-dessus permet d'avoir un volume disponible important entre les longerons longitudinaux 14 au-dessus des traverses 28, comme cela est visible sur les figures 1 à 3. En effet, le bogie 1 ne comprend pas d'éléments disposés entre les deux longerons 14 au-dessus du plan défini par les traverses 28. Ce volume va permettre le passage d'un plancher d'interconnexion abaissé, comme cela va être décrit ultérieurement. Il est entendu qu'une autre structure de bogie peut être envisagée, en dehors de l'espace 30 et des surfaces de butées 32 et 34, tant qu'un volume est laissé libre pour le passage de l'interconnexion. A titre d'exemple, le bogie peut être un bogie entraîné, c'est-à-dire dépourvu de moteur, ou toutes les roues du bogie ne sont pas nécessairement entraînées en rotation par un moteur.

Les extrémités 10 en regard des caisses 8 des deux voitures 6 adjacentes sont reçues par des suspensions secondaires 40 prévues sur les longerons longitudinaux 14, permettant un débattement vertical des caisses 8 par rapport au bogie 1.

Les deux caisses 8 sont articulées entre elles par le dispositif d'articulation 2 fixé aux deux caisses de sorte à former une liaison articulée entre les deux caisses permettant d'absorber les débattements entre les deux caisses 8. Plus particulièrement, le dispositif d'articulation 2 comprend par exemple un premier élément d'articulation 42 fixé à l'extrémité 10 de l'une des caisses 8, et un deuxième élément d'articulation 44 fixé à l'extrémité 10 de l'autre caisse 8, les premier et deuxième éléments d'articulation 42 et 44 étant reliés l'un à l'autre de sorte à former une liaison articulée entre les deux caisses. La liaison entre les deux éléments d'articulation 42 et 44 forme par exemple une liaison rotule 46 autorisant un débattement dans toutes les directions entre les caisses.

Les éléments d'articulation 42 et 44 sont fixés aux caisses 8 de sorte que le dispositif d'articulation 2 s'étend dans l'espace 30 entre les surfaces de butées 32 et 34, comme plus particulièrement visible sur la figure 4. Le dispositif d'articulation 2 et l'espace 30 sont dimensionnés de telle manière qu'un débattement existe selon la direction longitudinale. En d'autres termes, la distance séparant la première surface de butée 32 de la deuxième surface de butée 34 selon la direction longitudinale est supérieure à la dimension du dispositif d'articulation 2 selon la direction longitudinale.

Le premier élément d'articulation 42 comprend une surface de contre butée 48 s'étendant en regard de la première surface de butée 32 et le deuxième élément d'articulation 44 comprend une surface de contre butée 50 s'étendant en regard de la deuxième surface de butée 34. Les surfaces de contre butée portent en outre les moyens de fixation 52 à la caisse 8.

Ainsi, lorsque le véhicule ferroviaire est monté et lorsqu'il circule selon une première direction longitudinale de circulation allant de la première surface de butée 32 vers la deuxième surface de butée 34, le bogie 1 se déplace jusqu'à ce que la première surface de butée 32 entre en contact avec la surface de contre butée 48 du premier élément d'articulation 42, de sorte que le bogie 1 entraîne le déplacement des voitures 6 par l'intermédiaire du dispositif d'articulation 2 selon cette première direction longitudinale. De façon similaire, lorsque le véhicule ferroviaire se déplace selon une deuxième direction longitudinale de circulation, opposée à la première direction longitudinale, allant de la deuxième surface de butée 34 vers la première surface de butée, le bogie 1 se déplace jusqu'à ce que la deuxième surface de butée 34 entre en contact avec la surface de contre butée 50 du deuxième élément d'articulation 44, de sorte que le bogie 1 entraîne le déplacement des voitures 6 par l'intermédiaire du dispositif d'articulation 2 selon cette deuxième direction longitudinale.

Comme le dispositif d'articulation s'étend dans l'espace 30, ce dispositif d'articulation n'occupe pas non plus le volume libre entre les longerons longitudinaux 14 au-dessus des traverses 28. Ainsi, ce volume est laissé libre pour le passage d'un plancher d'interconnexion bas, comme cela va à présent être décrit.

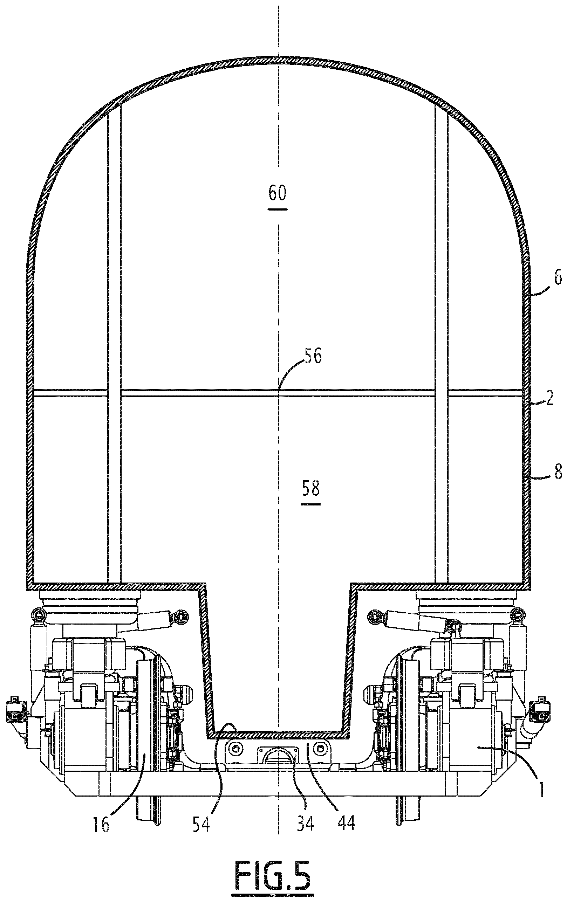

Chaque voiture 6 est une voiture à deux niveaux, comme cela est représenté sur la figure 5. Ainsi, chaque voiture 6 comprend un plancher inférieur 54 et un plancher supérieur 56, disposés l'un au-dessus de l'autre de sorte à définir un niveau inférieur 58 et un niveau supérieur 60. Le plancher inférieur 54 et le plancher supérieur 56 sont par exemple sensiblement plan et horizontaux de sorte que les voitures peuvent être franchies sans difficulté. En outre, les planchers inférieur 54 et supérieur 56 d'une voiture s'étendent au même niveau que les planchers inférieur 54 et supérieur 56 de l'autre niveau, c'est-à-dire que les planchers inférieur 54 et supérieur 56 de toutes les voitures s'étendent à la même hauteur, la hauteur des planchers supérieurs 56 étant supérieure à celle des planchers inférieurs 54. A titre d'exemple, le plancher inférieur 54 s'étend à une hauteur sensiblement comprise entre 530 et 550 mm et le plancher supérieur 56 s'étend à une hauteur sensiblement comprise entre 2380 et 2400 mm. Les hauteurs données ci-dessus sont dépendantes du gabarit dans lequel le véhicule doit s'inscrire.

Les voitures 6 sont reliées l'une à l'autre par un passage d'interconnexion 62 s'étendant sensiblement au droit du dispositif d'articulation 2 et permettant le passage d'une voiture à l'autre aux usagers du véhicule ferroviaire. A cet effet, le passage d'interconnexion 62 comprend un plancher inférieur, reliant les planchers inférieurs 54 des voitures 6 et s'étendant à la même hauteur que ces planchers, et un plancher supérieur, reliant les planchers supérieurs 56 des voitures 6 et s'étendant à la même hauteur que ces planchers. Ainsi, les utilisateurs du véhicule ferroviaire n'ont pas besoin de changer de niveau pour passer d'une voiture à l'autre, comme c'est usuellement le cas dans les véhicules ferroviaires à deux étages. En outre, le passage d'une voiture à l'autre par le niveau inférieur peut se faire sans franchir de marche ou de rampe puisque les planchers inférieurs s'étendent tous à la même hauteur. La circulation dans le véhicule ferroviaire est ainsi facilitée, en particulier pour les personnes à mobilité réduite.

La disposition du plancher inférieur du passage d'interconnexion 62 à la même hauteur que celle des planchers inférieurs 54 des voitures est rendue possible par l'agencement du bogie 1, qui présente un volume libre important entre les longerons longitudinaux 14. Ainsi, le plancher inférieur du passage d'interconnexion 62 peut s'étendre dans un plan confondu avec l'un des plans du bogie 1 et plus particulièrement avec un plan voisin du plan défini par les traverses 28, comme représenté sur la figure 4.

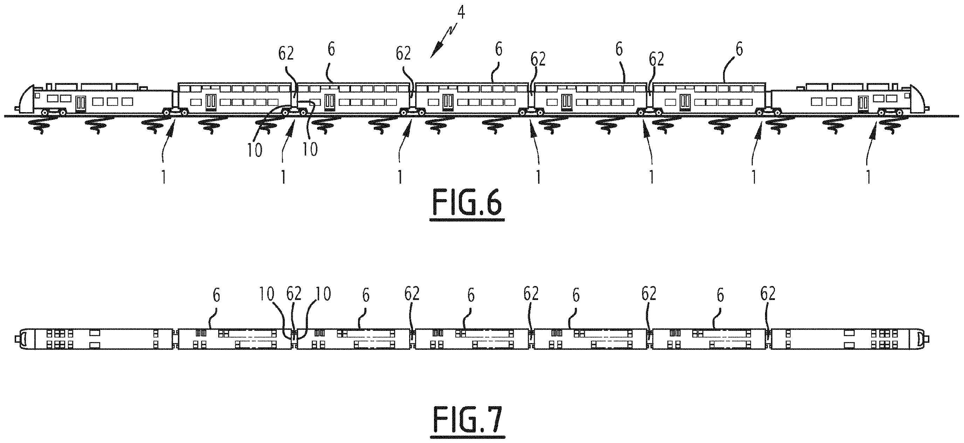

Le véhicule ferroviaire décrit ci-dessus peut être mis en œuvre, comme représenté sur les figures 6 et 7, avec plus de deux voitures 6 et avec une capacité d'accueil de voyageurs optimisée.

Selon le mode de réalisation représenté sur les figures 6 et 7, le véhicule ferroviaire comprend ainsi cinq voitures 6 à deux niveaux et deux voitures d'extrémités 64, comprenant chacune un niveau et un poste de pilotage. Les voitures à deux niveaux 6 sont reliées les unes aux autres par un passage d'interconnexion 62 tel que décrit ci-dessus. Un tel véhicule ferroviaire est apte à recevoir 600 passagers. En outre, le bogie est adapté pour des trains circulant à des vitesses élevées allant jusqu'à 200 km/h, voire au-delà, tout en ayant une structure permettant de recevoir un plancher inférieur abaissé, comme décrit précédemment.

Il est entendu que la structure du dispositif d'articulation 2 pourrait être différente de celle décrite ci-dessus, tant que ce dispositif d'articulation est apte à entrer en contact avec les surfaces de butée du bogie 1 pour que le bogie entraîne le déplacement des voitures du véhicule.

## Revendications

1. Véhicule ferroviaire comprenant au moins deux voitures (6) adjacentes, comprenant chacune une caisse (8) de voiture, lesdites caisses (8) étant supportées par un bogie (1) commun et étant articulées l'une à l'autre par un dispositif d'articulation (2) fixé aux deux caisses (6) de sorte à former une liaison articulée entre les deux caisses (6), le dispositif d'articulation (2) étant reçu dans un espace (30) du bogie (1), ledit espace (30) étant délimité selon une direction longitudinale par une première surface de butée (32) et par une deuxième surface de butée (34) s'étendant de part et d'autre dudit espace (30), **caractérisé en ce que** le dispositif d'articulation (2) s'étend en regard desdites première et deuxième surfaces de butée (32, 34) de sorte que, lorsque le véhicule ferroviaire circule dans une première direction longitudinale, la première surface de butée (32) entre en contact avec le dispositif d'articulation (2) de sorte que le déplacement du bogie (1) entraîne le déplacement des caisses (8) dans la première direction par l'intermédiaire du dispositif d'articulation (2), et, lorsque le véhicule ferroviaire circule dans une deuxième direction longitudinale opposée à la première direction, la deuxième surface de butée (34) entre en contact avec le dispositif d'articulation (2) de sorte que le déplacement du bogie (1) entraîne le déplacement des caisses (6) dans la deuxième direction longitudinale par l'intermédiaire du dispositif d'articulation (2),
le bogie (1) comprenant quatre pinces de freinage (26), chaque pince de freinage (26) étant disposée autour d'une partie d'une des roues (16) du bogie (1) afin de permettre le freinage du bogie (1) lorsque lesdites pinces (26) sont actionnées, les pinces (26) s'étendant deux à deux l'une en regard de l'autre selon la direction longitudinale de sorte que chaque pince (26) s'étend entre les roues (16) d'une des paires de roues, chaque pince (26) étant disposée du côté intérieur de la roue (16) autour de laquelle ladite pince (26) est disposée,
chaque voiture (6) comprenant un plancher inférieur (54) et un plancher supérieur (56), disposés l'un au-dessus de l'autre de sorte à définir un niveau inférieur (58) et un niveau supérieur (60),
les voitures (6) étant reliées l'une à l'autre par un passage d'interconnexion (62), ledit passage (62) s'étendant sensiblement au droit du dispositif d'articulation (2) et comprenant un plancher inférieur reliant les planchers inférieurs (54) des deux voitures (6) et un plancher supérieur reliant les planchers supérieurs (56) des deux voitures.

2. Véhicule ferroviaire selon la revendication 1, dans lequel le dispositif d'articulation (2) comprend un premier élément d'articulation (42) fixé à l'une des caisses (8) et un deuxième élément d'articulation (44) fixé à l'autre caisse (8), lesdits premier et deuxième éléments d'articulation (42, 44) étant reliés l'un à l'autre de sorte à former une liaison articulée entre les deux caisses (8), le premier élément d'articulation (42) s'étendant en regard de la première surface de butée (32) et le deuxième élément d'articulation (44) s'étendant en regard de la deuxième surface de butée (34).

3. Véhicule ferroviaire selon la revendication 1 ou 2, dans lequel le bogie (1) comprend un châssis (12) reposant sur quatre roues (16), une paire de roues (16) s'étendant de chaque côté du châssis (12), ledit châssis (12) supportant au moins un moteur (24) disposé sur un côté du châssis (12) et relié à au moins une roue (16) par des moyens de transmission, chaque roue (16) comportant un arbre de roue individuel (18).

4. Véhicule ferroviaire selon la revendication 3, dans lequel le bogie (1) comprend deux moteurs (24) disposés chacun sur un des côtés du châssis (12) de part et d'autre de l'espace (30) selon une direction transversale sensiblement perpendiculaire à la direction longitudinale, chaque moteur (24) étant relié à deux des roues (16) du bogie (1) par des moyens de transmission.

5. Véhicule ferroviaire selon l'une quelconque des revendications 3 à 4, dans lequel ledit châssis (12) du bogie (1) comprend deux longerons longitudinaux (14) disposés à l'extérieur des roues (16) et reliés entre eux par des traverses (28) possédant une partie centrale abaissée s'étendant au-dessous du plan défini par l'axe des roues (16).

6. Véhicule ferroviaire selon la revendication 1, dans lequel les planchers inférieurs (54) des voitures (6) et du passage d'interconnexion (62) s'étendent sensiblement à la même hauteur et les planchers supérieurs (56) des voitures (6) et du passage d'interconnexion (62) s'étendent sensiblement à la même hauteur, supérieure à la hauteur des planchers inférieurs (54).

7. Véhicule ferroviaire selon la revendication 6, dans lequel le plan des planchers inférieurs (54) est confondu avec un plan traversant le bogie (1).

## Patentansprüche

1. Schienenfahrzeug, aufweisend wenigstens zwei benachbarte Wagen (6), von denen jeder einen Wagen-Gehäusekasten (8) aufweist, wobei die Gehäusekästen (8) von einem gemeinsamen Drehgestell (1) getragen sind und zueinander gelenkig sind über eine Gelenkvorrichtung (2), die an beiden Gehäusekästen (6) befestigt ist, um eine Gelenkverbindung zwischen den beiden Gehäusekästen (6) zu bilden, wobei die Gelenkvorrichtung (2) in einem Raum (30) des Drehgestells (1) aufgenommen ist, wobei der Raum (30) entlang einer Längsrichtung begrenzt ist durch eine erste Anschlagfläche (32) und durch eine zweite Anschlagfläche (34), die sich beiderseits des Raums (30) erstrecken, **dadurch gekennzeichnet, dass** die Gelenkvorrichtung (2) sich gegenüber der ersten und der zweiten Anschlagfläche (32, 34) erstreckt, sodass, wenn das Schienenfahrzeug in einer ersten Längsrichtung fährt, die erste Anschlagfläche (32) in Kontakt mit der Gelenkvorrichtung (2) tritt, sodass die Verlagerung des Drehgestells (1) die Verlagerung der Gehäusekästen (8) in die erste Richtung bewirkt mittels der Gelenkvorrichtung (2), und, wenn das Schienenfahrzeug in eine zweite Längsrichtung entgegengesetzt zu der ersten Richtung fährt, die zweite Anschlagfläche (34) in Kontakt mit der Gelenkvorrichtung (2) tritt, sodass die Verlagerung des Drehgestells (1) die Verlagerung der Gehäusekästen (6) in die zweite Längsrichtung bewirkt mittels der Gelenkvorrichtung (2),
wobei das Drehgestell (1) vier Bremszangen (26) aufweist, wobei jede Bremszange (26) um einen Teil eines der Räder (16) des Drehgestells (1) angeordnet ist, um das Bremsen des Drehgestells (1) zu ermöglichen, wenn die Zangen (26) betätigt werden, wobei sich die Zangen (26) paarweise einander gegenüber erstrecken entlang der Längsrichtung, sodass jede Zange (26) sich zwischen den Rädern (16) eines der Paare von Rädern erstreckt, wobei jede Zange (26) auf der Innenseite des Rades (16) angeordnet ist, um welches die Zange (26) angeordnet ist,
wobei jeder Wagen (6) einen unteren Boden (54) und einen oberen Boden (56) aufweist, die übereinander angeordnet sind, um ein unteres Niveau (58) und ein oberes Niveau (60) zu definieren,
wobei die Wagen (6) miteinander verbunden sind durch eine Verbindungspassage (62), wobei die Passage (62) sich im Wesentlichen direkt auf Höhe der Gelenkvorrichtung (2) erstreckt und aufweist einen unteren Boden, der die unteren Böden (54) der beiden Wagen (6) verbindet, und einen oberen Boden, der die oberen Böden (56) der beiden Wagen (6) verbindet.

2. Schienenfahrzeug gemäß Anspruch 1, wobei die Gelenkvorrichtung (2) aufweist ein erstes Gelenkelement (42), das an einem der Gehäusekästen (8) befestigt ist, und ein zweites Gelenkelement (44), das an dem anderen Gehäusekasten (8) befestigt ist, wobei das erste und das zweite Gelenkelement (42, 44) miteinander verbunden sind, um eine Gelenkverbindung zwischen den beiden Gehäusekästen (8) zu bilden, wobei das erste Gelenkelement (42) sich gegenüber der ersten Anschlagfläche (32) erstreckt und das zweite Gelenkelement (44) sich gegenüber der zweiten Anschlagfläche (34) erstreckt.

3. Schienenfahrzeug gemäß Anspruch 1 oder 2, wobei das Drehgestell (1) ein Chassis (12) aufweist, das auf vier Rädern (16) liegt, wobei ein Paar von Rädern (16) sich auf jeder Seite des Chassis (12) erstreckt, wobei das Chassis (12) wenigstens einen Motor (24) trägt, der auf einer Seite des Chassis (12) angeordnet ist und mit wenigstens einem Rad (16) verbunden ist mittels Getriebemitteln, wobei jedes Rad (16) eine individuelle Radachse (18) aufweist.

4. Schienenfahrzeug gemäß Anspruch 3, wobei das Drehgestell (1) zwei Motoren (24) aufweist, die jeweils auf einer der Seiten des Chassis (12) beiderseits des Raums (30) angeordnet sind entlang einer Querrichtung im Wesentlichen senkrecht zu der Längsrichtung, wobei jeder Motor (24) mit zwei der Räder (16) des Drehgestells (1) verbunden ist mittels Getriebemitteln.

5. Schienenfahrzeug gemäß irgendeinem der Ansprüche 3 bis 4, wobei das Chassis (12) des Drehgestells (1) zwei Längsträger (14) aufweist, die auf der Außenseite der Räder (16) angeordnet sind und miteinander verbunden sind mittels Traversen (28), die einen zentralen, abgesenkten Teil haben, der sich unterhalb der Ebene erstreckt, die von der Achse der Räder (16) definiert ist.

6. Schienenfahrzeug gemäß Anspruch 1, wobei die unteren Böden (54) der Wagen (6) und der Verbindungspassage (62) sich im Wesentlichen auf der gleichen Höhe erstrecken und die oberen Böden (56) der Wagen (6) und der Verbindungspassage (62) sich im Wesentlichen auf der gleichen Höhe erstrecken, die größer ist als die Höhe der unteren Böden (54).

7. Schienenfahrzeug gemäß Anspruch 6, wobei die Ebene der unteren Böden (54) mit einer Querebene des Drehgestells (1) zusammenfällt.

## Claims

1. A railway vehicle comprising at least two adjacent carriages (6), each comprising a carriage body (8), said bodies (8) being supported by a common bogie (1) and being articulated with each other by an articulation device (2) attached to both bodies (6) so as to form an articulation connection between both bodies (8), the articulation device (2) being received in a space (30) of the bogie (1), said space (30) being delimited along a longitudinal direction by a first abutment surface (32) and by a second abutment surface (34) extending on either side of said space (30), **characterized in that** the articulation device (2) extends facing said first and second abutment surfaces (32, 34) so that, when the railway vehicle moves in a first longitudinal direction, the first abutment surface (32) comes into contact with the articulation device (2) so that the displacement of the bogie (1) causes displacement of the bodies (8) in the first direction via the articulation device (2), and, when the railway vehicle moves in a second longitudinal direction opposite to the first direction, the second abutment surface (34) comes into contact with the articulation device (2) so that the displacement of the bogie (1) causes displacement of the bodies (6) in the second longitudinal direction via the articulation device (2),
the bogie (1) comprising four braking clamps (26), each braking clamp (26) being positioned around a portion of one of the wheels (16) of the bogie so as to allow braking of the bogie (1) when said clamps (26) are actuated, the clamps (26) extending pair wise facing each other along the longitudinal direction so that each clamp (26) extends between the wheels (16) of one of the pairs of wheels, each clamp (16) being positioned on the inner side of the wheel (16) around which said clamp (26) is positioned,
each carriage (6) comprises a lower flooring (54) and an upper flooring (56), positioned above each other so as to define a low level (58) and an upper level (60),
the carriages (6) being connected to each other through an interconnection passage (62), said passage (62) substantially extending upright to the articulation device (2) and comprising a low flooring connecting the lower floorings (54) of both carriages (6) and an upper flooring connecting the upper floorings (56) of both carriages.

2. The railway vehicle according to claim 1, wherein the articulation device (2) comprises a first articulation element (42) attached to one of the bodies (8) and a second articulation element (44) attached to the other body (8), said first and second articulation elements (42, 44) being connected to each other so as to form an articulation connection between both bodies (8), the first articulation element (42) extending facing the first abutment surface (32) and the second articulation element (44) extending facing the second abutment surface (34).

3. The railway vehicle according to claim 1 or 2, wherein the bogie (1) comprises a chassis (12) lying on four wheels (16), a pair of wheels (16) extending on each side of the chassis (12), said chassis (12) supporting at least one motor (24) positioned on one side of the chassis (12) and connected to at least one wheel (16) by transmission means, each wheel (16) including an individual wheel shaft (18).

4. The railway vehicle according to claim 3, wherein the bogie (1) comprises two motors (24) each positioned on one of the sides of the chassis (12) on either side of the space (30) along a transverse direction substantially perpendicular to the longitudinal direction, each motor (24) being connected to two of wheels (16) of the bogie (1) by transmission means.

5. The railway vehicle according to any of claims 3 to 4, wherein said chassis (12) of the bogie (1) comprises two longitudinal members (14) positioned on the outside of the wheels (16) and connected together through crosspieces (28) having a central lowered portion extending below the plane defined by the axis of the wheels (16).

6. The railway vehicle according to claim 1, wherein the lower floorings (54) of the carriages (6) and of the interconnection passage (62) extend substantially at the same height and the upper floorings (56) of the carriages (6) and of the interconnection passage (62) substantially extend at the same height, greater than the height of the lower floorings (54).

7. The railway vehicle according to claim 6, wherein the plane of the lower floorings (54) coincides with a plane crossing the bogie (1).
